# EUROPEAN PATENT SPECIFICATION

(11) **EP 3 849 640 B1**
(45) Date of publication and mention of the grant of the patent: **10.12.2025**
(21) Application number: 19773679.6
(22) Date of filing: 11.09.2019
(51) Int. Cl.: A61M 5/31, A61M 39/16, A61J 1/06, A61M 5/28, A61M 5/00, A61M 5/24

(54) **COLLAPSIBLE SYRINGE BARREL DISINFECTION CAP**
ZUSAMMENFALTBARE SPRITZENKÖRPER DESINFEKTIONSKAPPE
CYLINDRE DE SERINGUE PLIABLE CAPOUCHON DE DÉSINFECTION

(30) Priority: 14.09.2018 US 201862731174 P
(43) Date of publication of application: 21.07.2021
(73) Proprietor: Becton, Dickinson and Company, Franklin Lakes, NJ 07417-1880 (US)
(72) Inventor: RYAN, Kevin M., Whitehouse Station, New Jersey 08889 (US); TRIPATHI, Sandeep, Morris Plains, New Jersey 07950 (US); PARTHAN, Vinay, Montville, New Jersey 07045 (US)
(74) Representative: dompatent
(86) International application number: PCT/US2019/050543
(87) International publication number: WO 2020/055960

(56) References cited:
- WO-A1-83/00882
- WO-A1-86/05989
- AU-A1- 2016 219 664
- US-A- 3 301 293
- US-A1- 2003 167 041
- US-A1- 2013 281 940
- US-B2- 7 232 419

## Description

### TECHNICAL FIELD

The present disclosure generally relates to syringe assemblies and particularly to pre-filled collapsible syringe assemblies having a locking element, and a collar extending from a distal wall of the barrel and surrounding a elongate tip for connection to a male or female connector. The present disclosure also generally relates to syringe assemblies and particularly to pre-filled collapsible syringe assemblies having an absorbent material with a disinfectant or antimicrobial agent to ensure adherence to aseptic techniques for use in flush procedures for vascular access devices (VAD's).

### BACKGROUND

Vascular access devices (VADs) are commonly used therapeutic devices, which include intravenous (IV) catheters, syringes, extension sets, stop cocks, tubing, high pressure extension tubing, and needleless access devices. The operation of VADs is often compromised or completely prevented by the occurrence of thrombus formation. Thrombosis is the development of a blood clot within a vessel and/or vascular access device. If not properly maintained or if exposed to a non-sterile environment, the VADs can become contaminated, sealed with blood clots or spread infection. To ensure VADs are used properly and do not become sealed or infected, protocols to ensure sterile practice have been developed. These protocols include sterilizing the VAD and flushing the catheter with a flush solution. Catheters are flushed using syringe assemblies filled with various fluids. In some cases, different fluids are injected sequentially in accordance with the protocol. For example, a saline solution followed by an anticoagulant such as heparin. The size of the syringe used to flush intravenous (I.V.) lines varies by various factors including the size and length of the catheter. Typically syringes of 1ml, 3ml, 5ml and 10ml volume are used. VAD protocols usually recommend flush procedures be performed after catheter placement, before fluid infusion, and before and after drug administration, blood sampling, transfusions and parenteral nutrition. The goal of these flush procedures is to confirm catheter patency, avoid drug incompatibilities, ensure the complete drug dose administration, prevent thrombus formation and minimize the risk of blood stream infections.

Conventional flush syringes have a barrel with a luer tip at one end which is exposed to the non-sterile environment once the syringe tip is removed from packaging thus providing an opportunity for undesired contamination.

Current "recommended practice" for aseptic IV line maintenance and IV drug delivery practices require adherence to a stepwise process referred to as "SASH." During the first step of the process, the clinician cleans/disinfects (generally with an alcohol swab) the VAD connector. Second, a syringe containing saline is used to flush the IV line or catheter (**S**aline flush), and then the VAD connector is disinfected a second time. Third, the fluid or pharmaceutical therapy is administered through the IV line or catheter (**A**dminister therapy), the VAD connector is disinfected a third time, followed by a second **S**aline flush step. The final step, which is dependent upon the patient's need and institutional poilcy, is a final disinfection of the VAD connector followed by a **H**eparin lock step, where a small amount of heparin is injected into the IV line or catheter to prevent the formation of thrombi or blood clots. A separate disinfecting cap may be used to sterile the hub of the VAD which after sterilization is performed the disinfecting cap is discarded. At the conclusion of this tedious stepwise process, the inlet port of the VAD connector is left exposed to the environment. This "recommended practice" requires disinfecting the VAD connector after each access makes IV line maintenance a very burdensome and time consuming process. Because the process is so cumbersome, clinicians very rarely implement this "recommended practice" in its entirety, and, thus, patients are exposed to the risk of contracting CRBSIs. Microorganisms populate exposed connector inlet surfaces, and, when the "recommended practice" is not adhered to, the microorganisms can enter the IV line during flushing. Furthermore, blood reflux into the IV line or catheter can cause clot formation inside the lines, and microorganisms from the connector inlet surfaces can colonize blood clots inside the lines and infect the patients during flushing.

There is a need, therefore, for a flush syringe assembly that promotes compliance with aseptic technique by eliminating the additional swabbing and disinfecting steps while reducing the number of separate flushing and disinfecting apparatuses used in current practice.

D1 (US 2013/281940 A1) discloses a bellows assembly for a fluid delivery system is a multi-component device that includes a cylindrical pressure jacket and a bellows syringe that is received within the pressure jacket. The bellows syringe includes a cap member and a bellows member. The bellows syringe in one embodiment is adapted to be secured to the pressure jacket by the cap member.

D2 (WO8605989 A1) discloses a device for use in surgical procedures formed of a concertina cartridge of a resilient plastics material. The cartridge has a nozzle, the throat of which is designed to receive by snap-click action, a projection formed internally of the chamber, to hold the cartridge in a collapsed, but resiliently releasable, state as it is compressed to that state under concertina action. D3 (US 7,232,419 B2) provides a container for a medical device and a lid therefor. The lid and the container are configured for an easy snap release using a cam-follower system. D3 also provides a method for using an assembly of the container, lid and a medical device within the container to maintain sterile connections. D7 (US 3,301,293 A) discloses a corrugated container with locking elements.

Related Art can also be found in AU 2016219664 A1, US 2003/167041 A1 and WO 83/00882 A1.

### SUMMARY

One aspect of the present disclosure pertains to a flush syringe assembly including a barrel having a corrugated side wall having an inside surface defining a chamber, an closed proximal end, a distal end including a distal wall with an elongate tip extending distally therefrom having a passageway therethrough in fluid communication with said chamber. The flush syringe assembly also includes a pre-selected amount of fluid in the chamber, a collar extending from the distal wall of the barrel and surrounding the elongate tip, and a locking element disposed on an exterior surface of the corrugated side wall of the barrel. The collar includes at least one side wall having an inside surface defining a compartment, an open distal end, a proximal end adjacent the distal wall of the barrel.

In one or more embodiments, the fluid is a flush fluid wherein the flush fluid may be saline, heparin, water or a combination thereof.

In one or more embodiments, the compartment of the collar surrounds the elongate tip.

In one or more embodiments, the inside surface of the collar having a plurality of threads. In one or more embodiments, the plurality of threads are adapted for connection to a female luer connector. The female luer connector may be needle-free connectors, stopcocks, or hemodialysis connectors.

In one or more embodiments, the exterior surface of the collar having a plurality of threads. In one or more embodiments, the plurality of threads are adapted for connection to a male luer connector. The male luer connector may be an intravenous tubing end.

In one or more embodiments, the locking element minimizes reflux of solution in the passageway.

According to the invention, the locking element has at least one protrusion and at least one corresponding cavity. The at least one protrusion may be disposed on the one segment of the corrugated side wall of the barrel and the at least one corresponding cavity is disposed on an opposing segment of the corrugated side wall of the barrel. In one or more embodiments, the locking element is arranged to be manually activated by a user after the protrusion engages to the corresponding cavity after the fluid has been expelled from the chamber of the barrel.

In one or more embodiments, the locking element has detents. In yet another embodiment, the locking element may be a snap fit element to connect one segment of the corrugated side wall of the barrel to an opposing segment of the corrugated side wall of the barrel.

According to the invention, the locking element provides feedback to the user to confirm delivery of a desired volume of fluid from the chamber. The feedback may be tactile, visual or audible.

In one or more embodiments, a removable seal is disposed on the open distal end of the collar. The removable seal may be a peelable seal. In one or more embodiments, the removable seal is an aluminum or multi-layer polymer film peel back top. In one or more embodiments, the removable seal is heat-sealed or induction sealed to the open distal end of the collar to retain the absorbent material within the compartment of the collar.

Another aspect of the present disclosure pertains to a flush syringe assembly including a barrel having a corrugated side wall having an inside surface defining a chamber, an closed proximal end, a distal end including a distal wall with an elongate tip extending distally therefrom having a passageway therethrough in fluid communication with said chamber. The flush syringe assembly also includes a pre-selected amount of fluid in the chamber, a collar extending from the distal wall of the barrel and surrounding the elongate tip an absorbent material disposed in the compartment of the collar, a disinfectant or antimicrobial agent; and a locking element disposed on an exterior surface of the corrugated side wall of the barrel. The collar includes at least one side wall having an inside surface defining a compartment, an open distal end, a proximal end adjacent the distal wall of the barrel.

In one or more embodiments, the fluid is a flush fluid wherein the flush fluid may be saline, heparin, water or a combination thereof.

In one or more embodiments, the compartment of the collar surrounds the elongate tip.

In one or more embodiments, the disinfectant or antimicrobial agent is selected from the group consisting of isopropyl alcohol, ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof. The disinfectant or antimicrobial agent may be a fluid or a gel.

In one or more embodiments, the inside surface of the collar having a plurality of threads. In one or more embodiments, the plurality of threads are adapted for connection to a female luer connector. The female luer connector may be needle-free connectors, stopcocks, or hemodialysis connectors.

In one or more embodiments, the exterior surface of the collar having a plurality of threads. In one or more embodiments, the plurality of threads are adapted for connection to a male luer connector. The male luer connector may be an intravenous tubing end.

In one or more embodiments, the absorbent material is a foam or a sponge. The foam may be a polyurethane foam.

In one or more embodiments, the locking element minimizes reflux of solution in the passageway.

In one or more embodiments, the locking element has at least one protrusion and at least one corresponding cavity. The at least one protrusion may be disposed on the one segment of the corrugated side wall of the barrel and the at least one corresponding cavity is disposed on an opposing segment of the corrugated side wall of the barrel. In one or more embodiments, the locking element is arranged to be manually activated by a user after the protrusion engages to the corresponding cavity after the fluid has been expelled from the chamber of the barrel.

In one or more embodiments, the locking element has detents. In yet another embodiment, the locking element may be a snap fit element to connect one segment of the corrugated side wall of the barrel to an opposing segment of the corrugated side wall of the barrel.

In one or more embodiments, the locking element provides feedback to the user to confirm delivery of a desired volume of fluid from the chamber. The feedback may be tactile, visual or audible.

In one or more embodiments, a removable seal is disposed on the open distal end of the collar. The removable seal may be a peelable seal. In one or more embodiments, the removable seal is an aluminum or multi-layer polymer film peel back top. In one or more embodiments, the removable seal is heat-sealed or induction sealed to the open distal end of the collar to retain the absorbent material within the compartment of the collar.

Yet another aspect of the present disclosure pertains to a method of flushing, disinfecting and administering a fluid to a vascular access device comprising providing a flush syringe assembly having a barrel including a corrugated side wall having an inside surface defining a chamber, an closed proximal end, a distal end including a distal wall with an elongate tip extending distally therefrom having a passageway therethrough in fluid communication with said chamber; a pre-selected amount of fluid in the chamber; a collar extending from the distal wall of the barrel and surrounding the elongate tip, the collar including at least one side wall having an inside surface defining a compartment, an open distal end, a proximal end adjacent the distal wall of the barrel; an absorbent material disposed in the compartment of the collar; a disinfectant or antimicrobial agent; and a locking element disposed on an exterior surface of the corrugated side wall of the barrel; providing a vascular access device having a proximal end, a distal end and a passageway therethrough, said proximal end having a luer tip in fluid communication with said passageway; placing said distal end of said vascular access device in a blood vessel of a patient; engaging said collar of the flush syringe assembly with said luer tip of said vascular access device to compress the absorbent material allowing the disinfectant or antimicrobial agent to contact the vascular access device; applying force to said flush syringe assembly to deform and collapse the corrugated side wall of the barrel so that said fluid in said chamber flows through said passageway into said vascular access device; continuing to apply force to the proximal end of the barrel until the locking element engages; and retaining the flush syringe assembly to the vascular access device.

### BRIEF DESCRIPTION OF THE DRAWINGS

FIG. 1 illustrates a perspective view of a collapsible syringe in accordance with one or more embodiments of the present disclosure;
FIG. 2 illustrates a cross-sectional side view of a collapsible syringe in an initial stated in accordance with one or more embodiments of the present disclosure;
FIG. 3 illustrates a cross-sectional side view of a collapsible flush syringe of Fig. 2 in a collapsed and locked state;
FIG. 4 illustrates a perspective view of a collapsible flush syringe with a collar in accordance with one or more alternate embodiments of the present disclosure;
FIG. 5 illustrates a cross-sectional side view of a collapsible flush syringe with a collar and disinfectant-loaded swab in an initial stated in accordance with one or more alternate embodiments of the present disclosure; and
FIG. 6 illustrates a cross-sectional side view of a collapsible flush syringe of Fig. 5 in a collapsed and locked state.

### DETAILED DESCRIPTION

Before describing several exemplary embodiments of the present disclosure, it is to be understood that the disclosure is not limited to the details of construction or process steps set forth in the following description. The disclosure is capable of other embodiments and of being practiced or being carried out in various ways.

With respect to terms used in this disclosure, the following definitions are provided.

Reference to "flush syringe assembly" includes syringes that are indicated for use in the flushing of VADs. The practice of flushing ensures and maintains catheter patency and helps prevent the mixing of incompatible pharmaceuticals.

In this disclosure, a convention is followed wherein the distal end of the device is the end closest to a patient and the proximal end of the device is the end away from the patient and closest to a practitioner.

As used herein, the use of "a," "an," and "the" includes the singular and plural.

As used herein, the term "catheter related bloodstream infection" or "CRBSI" refers to any infection resulting from the presence of a catheter or IV line.

As used herein, the term "microorganism" refers to a microbe or organism that is unicellular or lives in a colony of cellular organisms. Microorganisms are very diverse; they include, but are not limited to bacteria, fungi, archaea, and protozoans. Microorganisms are often the cause of CRBSIs. The most common microorganisms associated with CRBSIs include, but are not limited to, Staphylococcus aureus and epidermis, Enterococcus faecalis, Escherichia coli, Pseudomonas aeruginosa, and Candida albicans.

As used herein, the terms "antimicrobial agent" or "antimicrobial" refers to substances that kill or inhibit the growth of microorganisms such as bacteria, fungi, archaea, or protozoans. Antimicrobial agents either kill microbes, or prevent the growth of microbes.

As used herein, the term "disinfectant" refers to antimicrobial substances that are used on non-living objects or outside the body, e.g., on the skin.

As used herein, the term "absorbent material" refers to a material having capacity or tendency to absorb or soak up another substance. In one or more embodiments, the absorbent material has a tendency to absorb a disinfectant or antimicrobial. Absorbent materials may include sponges, absorbent cottons.

The term "deformable" refers to a wall or container that is structured to be flexible enough to collapse at least partially into the inner chamber under manual depression. The shape and extent of the deformation will vary with the various configurations of the inner chamber and deformable barrel.

As used herein, the term "Luer connector" refers to a connection collar that is the standard way of attaching syringes, catheters, hubbed needles, IV tubes, etc. to each other. The Luer connector consists of male and female interlocking tubes, slightly tapered to hold together better with even just a simple pressure/twist fit. Luer connectors can optionally include an additional outer rim of threading, allowing them to be more secure. The Luer connector male end is generally associated with a flush syringe and can interlock and connect to the female end located on the VAD. A Luer connector comprises a distal end, a proximal end, an irregularly shaped outer wall, a profiled center passageway for fluid communication from the chamber of the barrel of a syringe to the hub of a VAD. A Luer connector also has a distal end channel that releasably attaches the Luer connector to the hub of a VAD, and a proximal end channel that releasably attaches the Luer connector to the barrel of a syringe.

Clinical best practice requires that clinicians disinfect the needleless connector with an alcohol swab, disinfectant cap, etc. before each flush, drug, and lock syringe, requiring the clinician to perform the disinfecting process multiple times for each catheter line access. In practice, there are low (40-45%) compliance rates to this disinfecting protocol. Embodiments of the present disclosure provide the advantage of increased and enforced compliance with the need to disinfect and flush needless connectors which ultimately reduces the chances of hospital acquired infections and complications. Use of embodiments of syringe assemblies disclosed in the present disclosure will require the clinician to open fewer packages and does not require the clinician to carry alcohol swabs. Moreover, use of embodiments of syringe assemblies disclosed in the present disclosure results in the combination of two existing steps (disinfection and flushing) into one step, thus simplifying workflow for the clinician. Embodiments of the present disclosure pertain to prefilled flush or lock syringes with an integrated absorbent material soaked with a disinfectant or antimicrobial agent that combines the two steps of disinfecting the hub and flushing the VAD into one, thus greatly improving compliance to best practices.

Provided are prefilled syringe assemblies which include a collapsible barrel having a closed proximal end and a distal tip, a collar that surrounds the distal syringe tip and extends from the distal wall of the syringe barrel to form a compartment, a locking element, and an integrated disinfection cap needleless connector tip. The collar also facilitates alignment of the syringe with a catheter hub or needle-free connector, as well as, reducing contamination of the syringe by preventing contact of the syringe tip with the surrounding non-sterile environment.

Also provided are prefilled syringe assemblies which include a collapsible barrel having a closed proximal end and a distal tip, a collar that surrounds the distal syringe tip and extends from the distal wall of the syringe barrel to form a compartment to house a disinfectant-loaded swab, a locking element and an integrated disinfection cap needleless connector tip. The collar also facilitates alignment of the syringe with a catheter hub or needle-free connector, as well as, reducing contamination of the syringe by preventing contact of the syringe tip with the surrounding non-sterile environment.

The present invention overcomes problems associated with known flushing techniques and flush devices by providing a single use pre-filled flush syringe assembly for VAD delivery that effectively scrubs the blood residue and other debris from deadspace located between the male or female luer connections, as well as other parts of the VAD. The device of the present disclosure may be used in medical applications such as IV flushing and disinfection for routine IV catheter patency maintenance. A single use pre-filled flush syringe assembly of the present disclosure overcomes problems found with the prior art by reducing the risk associated with contamination due to manually filling a syringe with solution from a vial. Other advantages of pre-filled flush syringe assembly of the present disclosure over prior art include the following: a) reduces the steps the clinician has to take when preforming routine IV Catheter patency maintenance by eliminating the steps of removing the syringe and applying a separate disinfection cap onto the connector; b) the flush syringe assembly of the present disclosure is capable of generating a secure connections with a receiving needleless vascular access connector; c) the flush syringe assembly of the present disclosure can be made using the blow, fill, seal process, which enables the creation of a sterile and protected luer connector; d) ease of use of the flush syringe assembly of the present disclosure; e) low part count because the flush syringe assembly of the present disclosure combines the separate functions of disinfection and flushing into one step using one device; f) low part complexity; and g) relatively compact design.

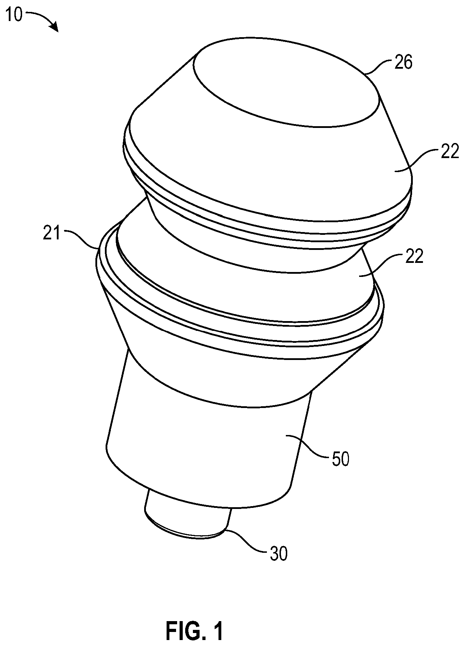

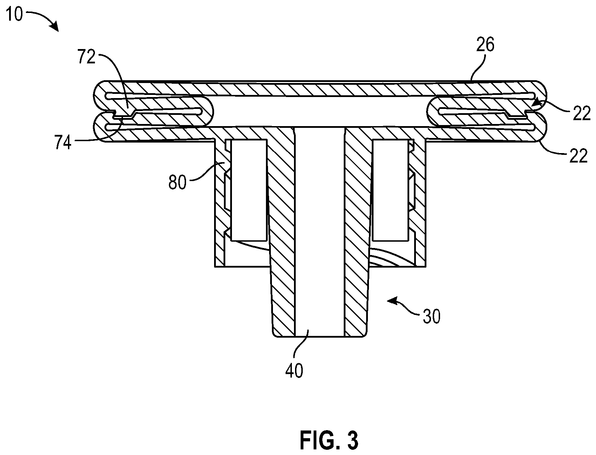

Figures 1-3 illustrate an exemplary flush syringe assembly 10 according to the present disclosure. Referring to Figures 1-3, a syringe assembly 10 according to the present disclosure generally comprises a manually deformable barrel 20 having a corrugated side wall 22 having an inside surface 23 defining a chamber 24 for retaining a fluid, an closed proximal end 26, a distal end 28 including a distal wall 29 with an elongate tip 30 extending distally therefrom having a passageway 40 therethrough in fluid communication with the chamber 24. In one or more embodiments, the collar 50 surrounds elongate tip 30 extending distally from the distal wall 29 of the barrel 20. The elongate tip 30 having a passageway 40 therethrough in fluid communication with the chamber 24, the collar extending from the distal wall 29 of the barrel to surround the elongate tip 30. In a further embodiment, the collar 50 surrounds an elongate tip adapted for connection to the hub of the vascular access devices, wherein the tip 30 is a Luer tip. The tip 30 may include a luer slip connection or a locking luer type collar concentrically surrounding the tip or within the tip. In one or more embodiments, the device may be used with ISO594-2 type fittings. The dimensions of the luer connection of the present invention can be made to comply with applicable standards and/or regulations, such as ISO standard 594.

In one or more embodiments, the corrugated side wall 22 includes a bendable wall in the shape of bellows or having corrugated or accordion-style walls. As used herein, the term "bellows" as used herein is used to describe a structure possessed by what are traditionally referred to as bellows or accordion-style walls. The term "bellows" also includes the alterable structure achieved by repeated corrugations or bends extending around the circumference or perimeter of a body. The term "bellows" also includes other deformable containers or flexible or semi-flexible fluid enclosures or containers that may hold fluid and function as a pump, such as diaphragm devices, springs, and the like. In one or more embodiments, the manually deformable barrel 20 includes a corrugated side wall 22 formed from an elastomeric material and has a spring constant that permits expansion and compression of the corrugated wall.

As shown in Fig. 1, the corrugated barrel may also include a thumbpress at the closed proximal end 26. The shape of the thumbpress can vary depending on the desired usage of the flush syringe assembly. The shape of the thumbpress may be round, square, rectangular, triangular, oval, pentagonal, hexagonal and cruciform.

The flush syringe assembly 10 also includes a pre-selected amount of fluid in the chamber. The syringe assembly 10 may be filled with flush solution using known methods. Additionally, the syringe assembly 10 may be provided pre-filled from the manufacturer or supplier. The flush solution may be any solution intended for flushing or maintaining performance of VAD's. In one or more embodiments, the flush solution is saline, heparin, water or a combination thereof. It is preferred that the flush solution be selected from the group consisting of saline flush solution and heparin lock flush solution. These solutions are known in the art and are readily available. An example of a saline flush solution includes, but is not limited to, 0.9% sodium chloride USP for injection. An example of a heparin lock flush solution includes but is not limited to 0.9% sodium chloride with 100 USP units of heparin sodium per mL or 10 USP units of heparin sodium per mL.

A collar 50 is disposed on the distal end 28 of the barrel 20 and extends from the distal wall 29 of the barrel 20 to form a compartment 52 that surrounds the elongate tip 30. The collar 50 includes at least one side wall 51 having an inside surface 53 defining compartment 52, an open distal end 54, a proximal end 56 adjacent the distal wall 29 of the deformable barrel 20.

In one or more embodiments, the inside surface of the collar has threads that have a size and pitch to engage a threadable segment of a male or female connector. Such connectors are generally and commonly used as catheter and other fluid-tight protective connectors in medical applications. In one or more embodiments, the inside surface 53 of the collar 50 include a plurality of threads 80. In one or more embodiments, the plurality of threads 80 are adapted for connection to a female luer connector. The female luer connector may be needle-free connectors, stopcocks, or hemodialysis connectors. The open distal end 54 of the collar 50 may comprise a plurality of threads 80 on the inside surface for attachment to a corresponding VAD. Elongated tip 30 may be adapted for connection to a hub of a vascular access device. In an alternate embodiment (not shown), the collar 50 may comprise a plurality of threads 80 on the exterior surface 55 of the collar for connection to a vascular access device. In one or more embodiments, the plurality of threads 80 are adapted for connection to a male luer connector. The male luer connector may be an intravenous tubing end.

In one or more embodiments, the shape of the collar 50 can vary. Collar 50 may have shapes including, but not limited to, a convex inner surface (for example a paraboloid), concave inner surface, with a straight profile (i.e., semi conical shape), or have the shape of a trapezoidal prism. The length of this extension from the main body of syringe and the degree of openness/ straightness of the profile (how wide the collar is at the end farthest from the syringe barrel) can vary.

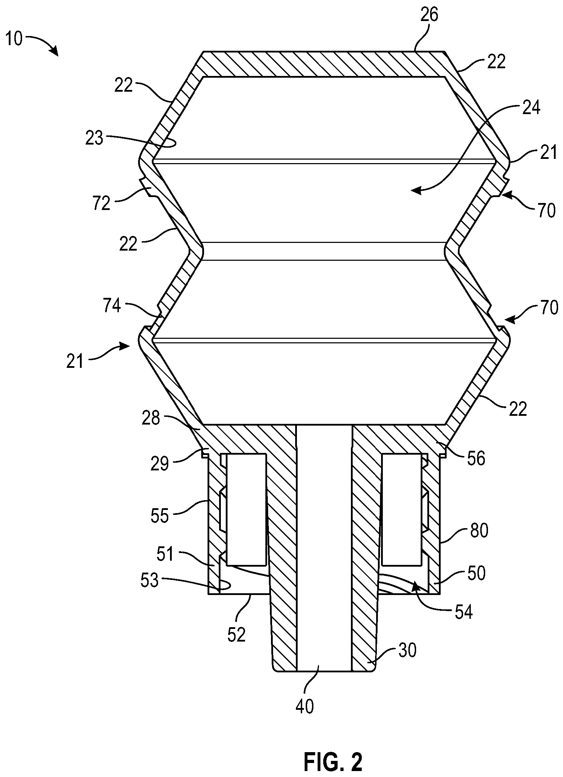

As shown in Fig. 2, the collar 50 surrounds elongate tip 30 adapted for connection to the hub of a vascular access device. In one or more embodiments, the elongate tip 30 is a Luer tip.

In one or more embodiments, the collar 50 may also comprise an aluminum lining adhered to the inside surface 53 of at least one side wall 51. The aluminum lining can provide a mechanism for ensuring compliance with aseptic conditions.

If necessary, cap or seal is removed from the distal end of the collar 50, exposing the tip 30. Once the connection of the syringe assembly 10 to the hub is completed, fluid communication from the barrel 20 of the syringe to the vascular access device can occur. Fluid is drawn from the barrel 20 through the passageway 40 through the hub and into the IV or catheter.

A locking element 70 disposed on an exterior surface 21 of the corrugated side wall 22 of the barrel 20 such that the barrel 20 locks when in a collapsed position at the end of a flush procedure.

The deformable barrel 20, collar 50 and locking element 70 may be made of thermoplastic elastomers, polyolefin, polyester, polycarbonate, polypropylene, polyethylene, glycol-modified polyethylene terephthalate, acrylonitrile butadiene styrene or other injection moldable or formable resin, natural rubber, synthetic rubber, thermoplastic materials, or other easily disposable and/or recyclable material and combinations thereof. Thermoplastic elastomers include, but are not limited to, polypropylene, polyethylene and the like. Materials should be chosen to be compatible with the solution, medicament and manufacturing process being used. It is envisioned that in one or more embodiments, the syringe assembly of the present disclosure may be made of a single material to facilitate recycling of the device.

In one or more embodiments, the fluid is a flush fluid. In one or more embodiments, the flush fluid may be saline, heparin, water or a combination thereof.

In one or more embodiments, the compartment of the collar surrounds the elongate tip.

The locking element 70 of the present disclosure minimizes, limits or prevents reuse of the device. In one or more embodiment, the locking element of the present disclosure minimizes, limits or prevents reflux of solution in the passageway. The locking element also provides confirmation to the user of solution delivery by providing feedback to the user to confirm delivery of a desired volume of fluid from the chamber. The feedback may be tactile, visual or audible. In one or more embodiments, as shown in Fig. 2, the locking element 70 includes at least one protrusion 72 and at least one corresponding cavity 74. In one or more embodiments, the at least one protrusion 72 is disposed on the one segment of the corrugated side wall 22 of the barrel and the at least one corresponding cavity 74 is disposed on an opposing segment of the corrugated side wall 22 of the barrel. In one or more embodiments, the locking element 70 is arranged to be manually activated by a user after the protrusion 72 engages to the corresponding cavity 74 after the fluid has been expelled from the chamber 24 of the barrel 20.

In one or more embodiments, the locking element 70 has detents. In yet another alternate embodiment, the locking element 70 may be a snap fit element to connect one segment of the corrugated side wall 22 of the barrel to an opposing segment of the corrugated side wall of the barrel. When the entire contents of the chamber 24 are expelled and the protrusion 72 is in contact and engages with the cavity 74 to locks the proximal end of the barrel to the distal end of the barrel.

In one or more embodiments, the locking element 70 provides feedback to the user to confirm delivery of a desired volume of fluid from the chamber. The feedback may be tactile, visual or audible.

In one or more embodiments, a removable seal or protective tip cap may be disposed on the open distal end 54 of the collar 50. The removable seal may be a peelable seal. In one or more embodiments, the removable seal may be an aluminum or multi-layer polymer film peel back top. The seal can be a plastic sealed aluminum, and can be chemically-resistant, light-blocking, non-permeable, or sterile. The removable seal prevents the prefilled flush solution from exiting the chamber of the barrel. In one or more embodiments, the removable seal is heat-sealed or induction sealed to the open distal end of the collar. In one or more embodiments, the removable seal comprises a moisture barrier.

In one or more embodiments, the syringe assembly 10 may also include a molded tip cap releasably connected to the distal end of the syringe assembly. The molded tip cap can be manually released by hand and is configured to be readily gripped by hand for removal.

The syringe assembly 10 of the present disclosure may be used in conjunction with a vascular access device. Another aspect of the present disclosure pertains to a method of flushing, disinfecting and administering a fluid to a vascular access device, wherein the user engages a flush syringe assembly 10 of the present disclosure having a barrel including a corrugated side wall having an inside surface defining a chamber, an closed proximal end, a distal end including a distal wall with an elongate tip extending distally therefrom having a passageway therethrough in fluid communication with said chamber; a pre-selected amount of fluid in the chamber; a collar extending from the distal wall of the barrel and surrounding the elongate tip, the collar including at least one side wall having an inside surface defining a compartment, an open distal end, a proximal end adjacent the distal wall of the barrel; and a locking element disposed on an exterior surface of the corrugated side wall of the barrel. The user obtains a vascular access device having a proximal end, a distal end and a passageway therethrough, the proximal end having a luer tip in fluid communication with said passageway. The user places the distal end of the vascular access device in a blood vessel of a patient. The user then engages the collar of the flush syringe assembly with the luer tip of said vascular access device. The user then applies force to the flush syringe assembly 10 to deform and collapse the corrugated side wall 22 of the barrel so that said fluid in said chamber flows through said passageway 40 into said vascular access device. The user continues to apply force to the proximal end of the barrel until the locking element 70 engages and retains the flush syringe assembly to the vascular access device. Once the connection of the syringe assembly 10 to the VAD is completed, fluid communication from the barrel 20 of the syringe to the vascular access device can occur. Fluid is drawn from the barrel 20 through the integral passageway 40 into the IV or catheter. At the end of the flush, the flexible, collapsible syringe barrel locks at the end position, and serves as a cap. The syringe assembly 10 is then left on the IV Connector to prevent microbial infection of the IV line. Therefore, the device of the present disclosure is an improvement over prior art in that it reduces the steps the clinician has to take when performing routine IV catheter patency maintenance. The device also allows practitioner to reduce the number of pieces of equipment/components required to perform flushing and maintaining sterility by combining the flushing feature of a flush syringe with the capabilities of a cap.

One advantage of the syringe assembly of the present disclosure is that the syringe assembly collapses to a configuration with minimal dead space and secures using the locking element 70. Another advantage of the present disclosure is that the deformable barrel 20 allows the user to sense the resistance in the fluid path, wherein increased resistance could allow the operator to detect resistance within the components of the syringe assembly or vascular access device.

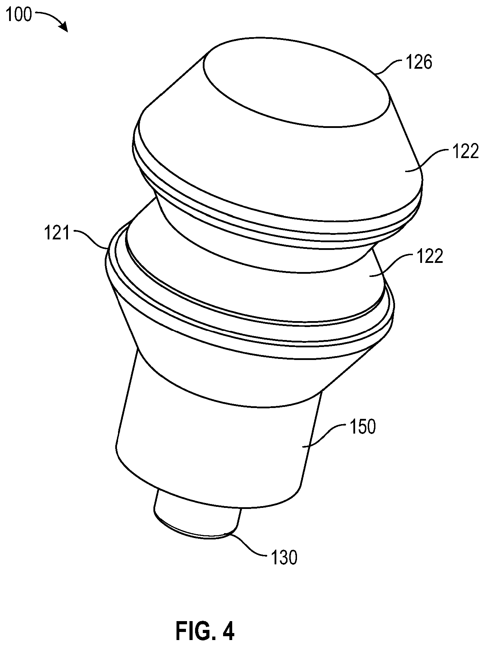

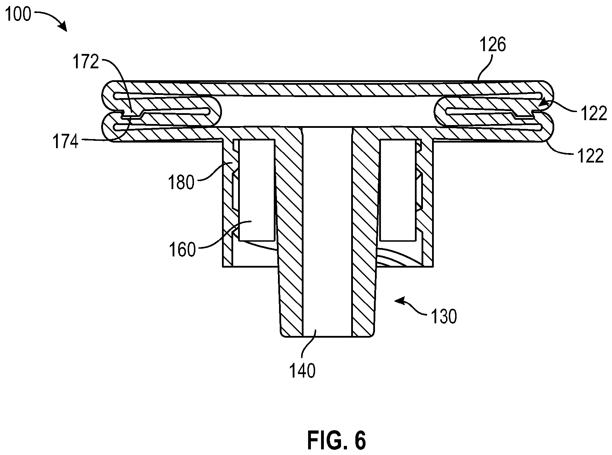

Figures 4-6 illustrate an exemplary flush syringe assembly 100 according to the present disclosure. Referring to Figures 4-6, a syringe assembly 100 according to the present disclosure generally comprises a manually deformable barrel 120 having a corrugated side wall 122 having an inside surface 123 defining a chamber 124 for retaining a fluid, an closed proximal end 126, a distal end 128 including a distal wall 129 with an elongate tip 130 extending distally therefrom having a passageway 140 therethrough in fluid communication with the chamber 124. In one or more embodiments, the collar 150 surrounds elongate tip 130 extending distally from the distal wall 129 of the barrel 120. The elongate tip 130 having a passageway 140 therethrough in fluid communication with the chamber 124, the collar extending from the distal wall 129 of the barrel to surround the elongate tip 130. In a further embodiment, the collar 150 surrounds an elongate tip adapted for connection to the hub of the vascular access devices, wherein the tip 130 is a Luer tip. The tip 130 may include a luer slip connection or a locking luer type collar concentrically surrounding the tip or within the tip. In one or more embodiments, the device may be used with ISO594-2 type fittings. The dimensions of the luer connection of the present invention can be made to comply with applicable standards and/or regulations, such as ISO standard 594.

In one or more embodiments, the corrugated side wall 122 includes a bendable wall in the shape of bellows or having corrugated or accordion-style walls. As used herein, the term "bellows" as used herein is used to describe a structure possessed by what are traditionally referred to as bellows or accordion-style walls. The term "bellows" also includes the alterable structure achieved by repeated corrugations or bends extending around the circumference or perimeter of a body. The term "bellows" also includes other deformable containers or flexible or semi-flexible fluid enclosures or containers that may hold fluid and function as a pump, such as diaphragm devices, springs, and the like. In one or more embodiments, the manually deformable barrel 120 includes a corrugated side wall 122 formed from an elastomeric material and has a spring constant that permits expansion and compression of the corrugated wall.

As shown in Fig. 4, the corrugated barrel may also include a thumbpress at the closed proximal end 126. The shape of the thumbpress can vary depending on the desired usage of the flush syringe assembly. The shape of the thumbpress may be round, square, rectangular, triangular, oval, pentagonal, hexagonal and cruciform.

The flush syringe assembly 100 also includes a pre-selected amount of fluid in the chamber. The syringe assembly 100 may be filled with flush solution using known methods. Additionally, the syringe assembly 100 may be provided pre-filled from the manufacturer or supplier. The flush solution may be any solution intended for flushing or maintaining performance of VAD's. In one or more embodiments, the flush solution is saline, heparin, water or a combination thereof. It is preferred that the flush solution be selected from the group consisting of saline flush solution and heparin lock flush solution. These solutions are known in the art and are readily available. An example of a saline flush solution includes, but is not limited to, 0.9% sodium chloride USP for injection. An example of a heparin lock flush solution includes but is not limited to 0.9% sodium chloride with 100 USP units of heparin sodium per mL or 10 USP units of heparin sodium per mL.

A collar 150 is disposed on the distal end 128 of the barrel 120 and extends from the distal wall 129 of the barrel 120 to form a compartment 152 that surrounds the elongate tip 130. The collar 150 includes at least one side wall 151 having an inside surface 153 defining compartment 152, an open distal end 154, a proximal end 156 adjacent the distal wall 129 of the deformable barrel 120.

In one or more embodiments, the inside surface of the collar has threads that have a size and pitch to engage a threadable segment of a male or female connector. Such connectors are generally and commonly used as catheter and other fluid-tight protective connectors in medical applications. In one or more embodiments, the inside surface 153 of the collar 150 include a plurality of threads 180. In one or more embodiments, the plurality of threads 180 are adapted for connection to a female luer connector. The female luer connector may be needle-free connectors, stopcocks, or hemodialysis connectors. The open distal end 154 of the collar 150 may comprise a plurality of threads 180 on the inside surface for attachment to a corresponding VAD. Elongated tip 130 may be adapted for connection to a hub of a vascular access device. In an alternate embodiment (not shown), the collar 150 may comprise a plurality of threads 180 on the exterior surface 155 of the collar for connection to a vascular access device. In one or more embodiments, the plurality of threads 180 are adapted for connection to a male luer connector. The male luer connector may be an intravenous tubing end.

In one or more embodiments, the shape of the collar 150 can vary. Collar 150 may have shapes including, but not limited to, a convex inner surface (for example a paraboloid), concave inner surface, with a straight profile (i.e., semi conical shape), or have the shape of a trapezoidal prism. The length of this extension from the main body of syringe and the degree of openness/ straightness of the profile (how wide the collar is at the end farthest from the syringe barrel) can vary.

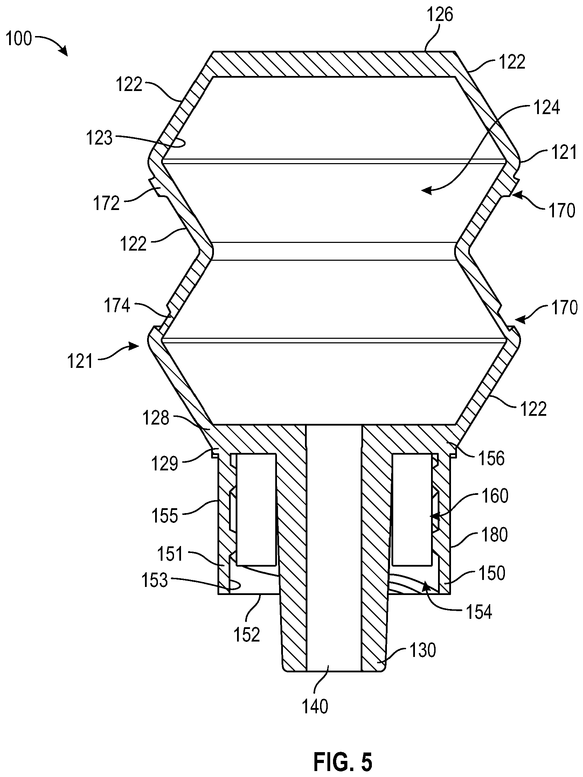

As shown in Fig. 5, the collar 150 surrounds elongate tip 130 adapted for connection to the hub of a vascular access device. In one or more embodiments, the elongate tip 130 is a Luer tip.

An absorbent material 160 is disposed and housed in the compartment 152 of the collar 150. Absorbent material 160 soaks up the disinfectant or antimicrobial agent that is housed within the compartment 152 of the collar 150. In one or more embodiments of the present disclosure, the absorbent material 160 can sit on top of the syringe tip without lateral support.

In one or more embodiments, the collar 150 may also comprise an aluminum lining adhered to the inside surface 153 of at least one side wall 151. The aluminum lining can prevent degradation of the disinfectant or antimicrobial agent, and can also provide a mechanism for ensuring compliance with aseptic conditions.

In one or more embodiments, the absorbent material 160 is a nonwoven material, foam or a sponge. In a specific embodiment, the foam is a polyurethane foam. In one or more embodiments, the absorbent material 160 may include one or more grooves that are sized and adapted to receive a male luer connector, a female luer connector or a hemodialysis connector. In one or more embodiments, the one or more grooves are disposed on the sidewall of the absorbent material 160 and are sized and adapted to receive a corresponding thread 180 disposed on the inside surface 153 of the collar 150. In one or more embodiments, the one or more grooves comprise one or more concentric cylindrical grooves which are cut into the absorbent material having a disinfectant or antimicrobial agent. In one or more embodiments, the concentric cylindrical groove corresponds with an end face of a stopcock.

In one or more embodiments, the concentric cylindrical groove accommodates the lumen of a stopcock to enhance the cleaning of the lumen of stopcock. In a specific embodiment, when stopcocks are screwed onto the threads 180 of collar, the concentric cylindrical groove of the absorbent material will be inserted into the lumen of the stopcock to sterilize the inner side of stopcocks. In one or more embodiments, the absorbent material 160 comprises one or more slits. In one or more embodiments, the one or more slits are sized and adapted to receive a male luer connector, a female luer connector or a hemodialysis connector. In a specific embodiment the absorbent material 160 is in the form of a foam plug. In one or more embodiments, the absorbent material 160 is under radial compression by the internal threads 180 to retain the absorbent material in the compartment 152 of the collar 150. In one or more embodiments, the absorbent material is retained in the compartment 152 of the collar 150 without radial compression by the internal threads 180.

Flush syringe assembly 100 can achieve disinfection when used on luer connectors by integrating disinfectant or antimicrobial agent in the compartment 152 of the collar 150. The disinfectant or antimicrobial agent can be directly included in the compartment 152 of the collar 150 or the disinfectant or antimicrobial agent can be absorbed into absorbent material 160 that fills the compartment of the collar. Flush syringe assembly 100 is designed to be compatible in interacting with various disinfectants. In one or more embodiments, the disinfectant or antimicrobial agent is selected from the group consisting essentially of isopropyl alcohol(IPA), ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof. In one or more embodiments, the disinfectant or antimicrobial agent may include variations of alcohol or chlorhexidine. In a specific embodiment, the disinfectant or antimicrobial agent comprises at least one of chlorhexidine gluconate and chlorhexidine diacetate. In one or more embodiments, the disinfectant or antimicrobial agent is a fluid or a gel.

In one or more embodiments, the absorbent material 160 in the compartment 152 of the collar 150 compresses upon connection to a male or female luer connector to disinfect the male or female luer connector.

The syringe assembly 100 having collar 150 and absorbent material 160 surrounding tip 130 that is rendered antimicrobial because the tip is surrounded by an absorbent material 160 that soaks up disinfectant or antimicrobial agent contained within compartment 152. The now antimicrobial tip 130 can be connected to a vascular access device. If necessary, cap or seal is removed from the distal end of the collar 150, exposing the tip 130. As the syringe assembly 100 is connected to the hub of a vascular access device, the disinfectant-loaded absorbent material 160 compresses creating friction. The disinfecting properties of the disinfectant or antimicrobial agent contained within the absorbent material 160 disposed in compartment 152 that has been absorbed by absorbent material 160, disinfect the hub of a vascular access device, thus ensuring compliance with aseptic technique. The friction created by the compression of the disinfectant-loaded absorbent material 160 is necessary to provide disinfection of the hub of a VAD. Once the connection of the syringe assembly 100 to the hub is completed, the hub is properly disinfected, and fluid communication from the barrel 120 of the syringe to the vascular access device can occur. Fluid is drawn from the barrel 120 through the passageway 140 through the hub and into the IV or catheter. Because of the presence of the collar 150 and disinfectant-loaded absorbent material 160, fluid communication through a vascular access device and into a patient is conducted under aseptic conditions without any additional swabbing steps and diligence on the part of the clinician.

In one or more embodiments, absorbent material 160 may include one or more openings or slits on the top surface to allow a needleless connector to go through to connect to the elongate tip 130. The absorbent material 160 will deform in way so as to create sufficient friction and scrubbing between the VAD connector and absorbent material 160, and to release the disinfectant as it gets compressed in order to disinfect the needleless connector surfaces. Following full engagement of the syringe assembly 100 and VAD connector, the fluid in the chamber 124 of the barrel 120 can be administered.

Absorbent material 160 can have a near cylindrical or hexagonal outer surface. The purpose of the hexagonal shape combined with the barrel 120 with the collar 150 is to provide a strong grip between the barrel 120 and absorbent material 160 and prevent disinfectant-loaded absorbent material 160 from rotating as the syringe assembly 100 gets twisted onto a VAD connector. The inner surface of the collar 150 can be cylindrical or hexagonal or any other geometry to increase friction between the absorbent material 160 and barrel 120. The opening at the top of the absorbent material 160 can be a single slit, two or more slits. In one or more embodiments, the thickness of absorbent material 160 can be adjusted to enable absorbance of sufficient amounts of disinfectant. Further, the inner surface of the absorbent material 160 may have various forms of cut-outs to allow for the disinfectant-loaded absorbent material 160 to buckle and fold on itself and allow the penetration of needleless connector. In an alternate embodiment, the absorbent material 160 fully rolls up on the sides of the needleless connector like a sleeve. The porosity of the absorbent material 160 can be different throughout the disinfectant-loaded absorbent material 160 (e.g. a radial or axial gradient or else) to allow for controlling the absorbance and release of the disinfectant. Additionally, the top surface can bear additional surface features for improving the scrubbing. A range of disinfectants (e.g. IPA, ethanol, chlorhexidine, etc.) at various concentrations can be loaded into the swab.

A locking element 170 disposed on an exterior surface 121 of the corrugated side wall 122 of the barrel 120 such that the barrel 120 locks when in a collapsed position at the end of a flush procedure.

The deformable barrel 120, collar 150 and locking element 170 may be made of thermoplastic elastomers, polyolefin, polyester, polycarbonate, polypropylene, polyethylene, glycol-modified polyethylene terephthalate, acrylonitrile butadiene styrene or other injection moldable or formable resin, natural rubber, synthetic rubber, thermoplastic materials, or other easily disposable and/or recyclable material and combinations thereof. Thermoplastic elastomers include, but are not limited to, polypropylene, polyethylene and the like. Materials should be chosen to be compatible with the solution, medicament and manufacturing process being used. It is envisioned that in one or more embodiments, the syringe assembly of the present disclosure may be made of a single material to facilitate recycling of the device.

In one or more embodiments, the fluid is a flush fluid. In one or more embodiments, the flush fluid may be saline, heparin, water or a combination thereof.

In one or more embodiments, the compartment of the collar surrounds the elongate tip.

In one or more embodiments, the disinfectant or antimicrobial agent is selected from the group consisting of isopropyl alcohol, ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof. The disinfectant or antimicrobial agent may be a fluid or a gel.

The locking element 170 of the present disclosure minimizes, limits or prevents reuse of the device. In one or more embodiment, the locking element of the present disclosure minimizes, limits or prevents reflux of solution in the passageway. The locking element also provides confirmation to the user of solution delivery by providing feedback to the user to confirm delivery of a desired volume of fluid from the chamber. The feedback may be tactile, visual or audible. In one or more embodiments, as shown in Fig. 5, the locking element 170 includes at least one protrusion 172 and at least one corresponding cavity 174. In one or more embodiments, the at least one protrusion 172 is disposed on the one segment of the corrugated side wall 122 of the barrel and the at least one corresponding cavity 174 is disposed on an opposing segment of the corrugated side wall 122 of the barrel. In one or more embodiments, the locking element 170 is arranged to be manually activated by a user after the protrusion 172 engages to the corresponding cavity 174 after the fluid has been expelled from the chamber 124 of the barrel 120.

In one or more embodiments, the locking element 170 has detents. In yet another alternate embodiment, the locking element 170 may be a snap fit element to connect one segment of the corrugated side wall 122 of the barrel to an opposing segment of the corrugated side wall of the barrel. When the entire contents of the chamber 124 are expelled and the protrusion 172 is in contact and engages with the cavity 174 to locks the proximal end of the barrel to the distal end of the barrel.

In one or more embodiments, the locking element 170 provides feedback to the user to confirm delivery of a desired volume of fluid from the chamber. The feedback may be tactile, visual or audible.

In one or more embodiments, a removable seal or protective tip cap may be disposed on the open distal end 154 of the collar 150. The removable seal may be a peelable seal. In one or more embodiments, the removable seal may be an aluminum or multi-layer polymer film peel back top. The seal can be a plastic sealed aluminum, and can be chemically-resistant, light-blocking, non-permeable, or sterile. The removable seal prevents the disinfectant or the antimicrobial agent from exiting the compartment of the collar and prevents the prefilled flush solution from exiting the chamber of the barrel. In one or more embodiments, the removable seal is heat-sealed or induction sealed to the open distal end of the collar to retain the absorbent material within the compartment of the collar. A seal will contain the disinfectant or antimicrobial agent within the chamber until the seal is removed and the syringe assembly 100 is connected to a vascular access device. The absorbent material 160 will soak up the disinfectant or antimicrobial agent and will disinfect the hub of a vascular access device upon connection. In one or more embodiments, the removable seal comprises a moisture barrier.

In one or more embodiments, the syringe assembly 100 may also include a molded tip cap releasably connected to the distal end of the syringe assembly. The molded tip cap can be manually released by hand and is configured to be readily gripped by hand for removal.

The syringe assembly 100 of the present disclosure may be used in conjunction with a vascular access device. Another aspect of the present disclosure pertains to a method of flushing, disinfecting and administering a fluid to a vascular access device, wherein the user engages a flush syringe assembly 100 of the present disclosure having a barrel including a corrugated side wall having an inside surface defining a chamber, an closed proximal end, a distal end including a distal wall with an elongate tip extending distally therefrom having a passageway therethrough in fluid communication with said chamber; a pre-selected amount of fluid in the chamber; a collar extending from the distal wall of the barrel and surrounding the elongate tip, the collar including at least one side wall having an inside surface defining a compartment, an open distal end, a proximal end adjacent the distal wall of the barrel; an absorbent material disposed in the compartment of the collar; a disinfectant or antimicrobial agent; and a locking element disposed on an exterior surface of the corrugated side wall of the barrel. The user obtains a vascular access device having a proximal end, a distal end and a passageway therethrough, the proximal end having a luer tip in fluid communication with said passageway. The user places the distal end of the vascular access device in a blood vessel of a patient. The user then engages the collar of the flush syringe assembly with the luer tip of said vascular access device to compress the absorbent material allowing the disinfectant or antimicrobial agent to contact the vascular access device. The user then applies force to the flush syringe assembly 100 to deform and collapse the corrugated side wall 122 of the barrel so that said fluid in said chamber flows through said passageway 140 into said vascular access device. The user continues to apply force to the proximal end of the barrel until the locking element 170 engages and retains the flush syringe assembly to the vascular access device. Once the connection of the syringe assembly 100 to the VAD is completed, fluid communication from the barrel 120 of the syringe to the vascular access device can occur. Fluid is drawn from the barrel 120 through the integral passageway 140 into the IV or catheter. Because of the presence of the absorbent material 160 in the collar 150, fluid communication through a vascular access device and into a patient is conducted under aseptic conditions without any additional swabbing steps and diligence on the part of the clinician. At the end of the flush, the flexible, collapsible syringe barrel locks at the end position, and serves as a disinfection cap. The syringe assembly 100 is then left on the IV Connector to prevent microbial infection of the IV line. The compartment of the collar has a disinfection solution that is able to keep the IV connector disinfected. Therefore, the device of the present disclosure is an improvement over prior art in that it reduces the steps the clinician has to take when preforming routine IV catheter patency maintenance. Specifically it eliminates the steps of removing the syringe and applying a separate disinfection cap onto the connector. The device also allows practitioner to reduce the number of pieces of equipment/components required to perform flushing and disinfection to skw count for the hospital by combining the flushing feature of a flush syringe with the disinfection capabilities of a disinfection cap.

One advantage of the syringe assembly of the present disclosure is that the syringe assembly collapses to a configuration with minimal dead space and secures using the locking element (70, 170). Another advantage of the present invention is that the deformable barrel (20, 120) allows the user to sense the resistance in the fluid path, wherein increased resistance could allow the operator to detect resistance within the components of the syringe assembly or vascular access device.

Blow, fill, seal technology is well known in the art and consists of generating containers that are formed, filled with liquid, and sealed in a continuous process under sterile or aseptic conditions. In one or more embodiments of the present disclosure, the syringe assembly (10,100) may be produced using a blow, fill seal, process, in which the delivery device is created in a single manufacturing step. Syringe assembly (10,100) may be manufactured in accordance with a blow-fill-seal technique of a character well understood by those skilled in the art. The concept of a blow-fill-seal process is that a container is formed, filled, and sealed as a unitary container in a continuous manner without human intervention in a sterile, enclosed area inside a machine. Blow-fill-seal manufacturing forms a closed container by extruding and forming a parison within a mold, filling the container and sealing the container in a single step. This manufacturing process enables the device to be produced in a single process. For example, pharmaceutical grade resin is extruded into a tube, which is then formed into a barrel. A mandrel is inserted into the newly formed barrel and filled. The barrel is then sealed, all inside a sterile, shrouded chamber. The syringe assembly (10,100) is then discharged to a non-sterile area for packaging and distribution. This blow-fill-seal technique comprises the continuous extrusion through an extruder head of a length of a parison in the form of a hollow tube between and through two co-acting first or main mold halves. The method includes the step of cutting off the parison below the extruder head and above the main mold halves to create an opening which allows a blowing and filling nozzle assembly to be moved downwardly into the opening in the parison for molding and thereafter filling a molded container. When the barrel portion of the container assembly is filled with the desired amount of liquid, the blowing and filling nozzle assembly is retracted from the opening in the parison. A separate pair of co-acting second or upper sealing mold halves are then moved together around the exposed length of parison to form and seal the upper portion of the barrel. The finished syringe assembly, completely formed, filled, and sealed as a unitary structure is then conveyed out of the molding apparatus.

In one or more embodiments, the elongate tip (30,130) and collar (50,150) are molded parts that are inserted onto the barrel (20,120) during the blow, fill, seal process. In another embodiment, the elongate tip (30,130) and collar (50,150) are molded directly to the barrel 20 using the blow, fill, seal process.

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised within the scope of the independent claim. The invention is defined by the appended claims.

## Claims

1. A flush syringe assembly comprising:
a barrel (20) including a corrugated side wall (22) having an inside surface (23) defining a chamber (24), a closed proximal end (26), a distal end (28) including a distal wall (29) with an elongate tip (30) extending distally therefrom having a passageway (40) therethrough in fluid communication with said chamber (24);
a pre-selected amount of fluid in the chamber (24);
a collar (50) extending from the distal wall (29) of the barrel (20) and surrounding the elongate tip (30), the collar (50) including at least one side wall (51) having an inside surface (53) defining a compartment (52), an open distal end (54), a proximal end (56) adjacent the distal wall (29) of the barrel (20); and
a locking element (70) disposed on an exterior surface (55) of the corrugated side wall (22) of the barrel (20);
**characterized in that**:
the locking element (70) comprises at least one protrusion (72) and at least one corresponding cavity (74), the at least one protrusion (72) is disposed on one segment of the corrugated side wall (22) of the barrel (20) and the at least one corresponding cavity (74) is disposed on an opposing segment of the corrugated side wall (22) of the barrel (20),
whereby the protrusion 72 is in contact and engages with the cavity 74 to lock the proximal end of the barrel to the distal end of the barrel, after the fluid has been expelled from the chamber 24 of the barrel 20
whereby the locking element (70) provides tactile, visual and audible feedback.

2. The flush syringe assembly of claim 1, wherein the fluid is a flush fluid.

3. The flush syringe assembly of claim 2, wherein the flush fluid is saline, heparin, water or a combination thereof.

4. The flush syringe assembly of claim 1, wherein the compartment (52) of the collar (50) surrounds the elongate tip (30).

5. The flush syringe assembly of claim 1, wherein the inside surface (23) of the collar (50) having a plurality of threads (80).

6. The flush syringe assembly of claim 5, wherein the plurality of threads are adapted for connection to a female luer connector.

7. The flush syringe assembly of claim 1, wherein the exterior surface (55) of the collar (50) having a plurality of threads (80).

8. The flush syringe assembly of claim 5, wherein the plurality of threads (80) are adapted for connection to a male luer connector.

9. The flush syringe assembly of claim 1, wherein the locking element (70) minimizes reflux of solution in the passageway (40).

10. The flush syringe assembly of claim 1, wherein the locking element (70) comprises detents.

11. The flush syringe assembly of claim 1, wherein the locking element (70) comprises a snap fit element to connect one segment of the corrugated side wall (22) of the barrel (20) to an opposing segment of the corrugated side wall (22) of the barrel (20).

12. The flush syringe assembly of claim 1, further comprising a removable seal on the open distal end (28) of the collar (50).

13. The flush syringe assembly of claim 12, wherein the removable seal is a peelable seal.

14. The flush syringe assembly of claim 12, wherein the removable seal comprises an aluminum or multi-layer polymer film peel back top.

15. The flush syringe assembly of claim 12, wherein the removable seal is heat-sealed or induction sealed to the open distal end (28) of the collar (50).

16. The flush syringe assembly of any of the claims 1 - 15 further comprising:
an absorbent material disposed in the compartment of the collar;
a disinfectant or antimicrobial agent

17. The flush syringe assembly of claim 16, wherein the disinfectant or antimicrobial agent is selected from the group consisting of isopropyl alcohol, ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof.

18. The flush syringe assembly of claim 16, wherein the disinfectant or antimicrobial agent is a fluid or a gel.

19. The flush syringe assembly of claim 16, wherein the absorbent material is a foam.

20. The flush syringe assembly of claim 19, wherein the foam is a polyurethane foam.

21. The flush syringe assembly of claim 16, wherein the absorbent material is a sponge.

## Patentansprüche

1. Spülspritzenanordnung, die aufweist:
einen Zylinder (20), der eine gewellte Seitenwand (22) mit einer Innenfläche (23), die eine Kammer (24) definiert, ein geschlossenes proximales Ende (26), und ein distales Ende (28) aufweist, das eine distale Wand (29) mit einer sich distal davon erstreckenden länglichen Spitze (30) aufweist, die einen Durchgang (40) dort hindurch aufweist, der in Fluidverbindung mit der Kammer (24) steht;
eine vorausgewählte Menge an Fluid in der Kammer (24);
einen Kragen (50), der sich von der distalen Wand (29) des Zylinders (20) aus erstreckt und die längliche Spitze (30) umgibt, wobei der Kragen (50) mindestens eine Seitenwand (51) mit einer Innenfläche (53), die eine Kammer (52) definiert, ein offenes distales Ende (54), und ein proximales Ende (56) angrenzend an die distale Wand (29) des Zylinders (20) aufweist; und
ein Verriegelungselement (70), das auf einer Außenfläche (55) der gewellten Seitenwand (22) des Zylinders (20) angeordnet ist;
**dadurch gekennzeichnet, dass**
das Verriegelungselement (70) mindestens einen Vorsprung (72) und mindestens einen entsprechenden Hohlraum (74) aufweist, wobei der mindestens eine Vorsprung (72) auf einem Segment der gewellten Seitenwand (22) des Zylinders (20) angeordnet ist und der mindestens eine entsprechende Hohlraum (74) auf einem entgegengesetzten Segment der gewellten Seitenwand (22) des Zylinders (20) angeordnet ist, wobei der Vorsprung (72) mit dem Hohlraum (74) in Kontakt ist und in diesen eingreift, um das proximale Ende des Zylinders am distalen Ende des Zylinders zu verriegeln, nachdem das Fluid aus der Kammer (24) des Zylinders (20) ausgestoßen wurde,
wobei das Verriegelungselement (70) ein taktiles, visuelles und akustisches Feedback bereitstellt.

2. Spülspritzenanordnung nach Anspruch 1, wobei das Fluid ein Spülfluid ist.

3. Spülspritzenanordnung nach Anspruch 2, wobei das Spülfluid Kochsalzlösung, Heparin, Wasser oder eine Kombination davon ist.

4. Spülspritzenanordnung nach Anspruch 1, wobei die Kammer (52) des Kragens (50) die längliche Spitze (30) umgibt.

5. Spülspritzenanordnung nach Anspruch 1, wobei die Innenfläche (23) des Kragens (50) eine Vielzahl von Gewinden (80) aufweist.

6. Spülspritzenanordnung nach Anspruch 5, wobei die Vielzahl von Gewinden zur Verbindung mit einem weiblichen Luer-Konnektor geeignet sind.

7. Spülspritzenanordnung nach Anspruch 1, wobei die Außenfläche (55) des Kragens (50) eine Vielzahl von Gewinden (80) aufweist.

8. Spülspritzenanordnung nach Anspruch 5, wobei die Vielzahl von Gewinden (80) zur Verbindung mit einem männlichen Luer-Konnektor geeignet sind.

9. Spülspritzenanordnung nach Anspruch 1, wobei das Verriegelungselement (70) den Rückfluss von Lösung in dem Durchgang (40) minimiert.

10. Spülspritzenanordnung nach Anspruch 1, wobei das Verriegelungselement (70) Arretierungen aufweist.

11. Spülspritzenanordnung nach Anspruch 1, wobei das Verriegelungselement (70) ein Schnappverschlusselement aufweist, um ein Segment der gewellten Seitenwand (22) des Zylinders (20) mit einem entgegengesetzten Segment der gewellten Seitenwand (22) des Zylinders (20) zu verbinden.

12. Spülspritzenanordnung nach Anspruch 1, die ferner ein entfernbares Siegel auf dem offenen distalen Ende (28) des Kragens (50) aufweist.

13. Spülspritzenanordnung nach Anspruch 12, wobei das entfernbare Siegel ein abziehbares Siegel ist.

14. Spülspritzenanordnung nach Anspruch 12, wobei das entfernbare Siegel eine abziehbare Oberschicht aus Aluminium oder mehrschichtigem Polymerfilm aufweist.

15. Spülspritzenanordnung nach Anspruch 12, wobei das entfernbare Siegel am offenen distalen Ende (28) des Kragens (50) heißversiegelt oder induktionsversiegelt ist.

16. Spülspritzenanordnung nach einem der Ansprüche 1-15, die ferner aufweist:
ein Absorptionsmaterial, das in der Kammer des Kragens angeordnet ist;
ein Desinfektionsmittel oder ein antimikrobielles Mittel.

17. Spülspritzenanordnung nach Anspruch 16, wobei das Desinfektionsmittel oder das antimikrobielle Mittel ausgewählt ist aus der Gruppe bestehend aus Isopropylalkohol, Ethanol, 2-Propanol, Butanol, Methylparaben, Ethylparaben, Propylparaben, Propy-Igallat, Butylhydroxyanisol (BHA), Butylhydroxytoluol, t-Butylhydrochinon, Chloroxylenol, Chlorohexidin, Chlorhexidindiacetat, Chlorhexidin-Gluconat, Povidon-Iod, Alkohol, Dichlorbenzylalkohol, Dehydroessigsäure, Hexetidin, Triclosan, Wasserstoffperoxid, kolloidales Silber, Benzethoniumchlorid, Benzalkoniumchlorid, Octenidin, Antibiotika und Mischungen davon.

18. Spülspritzenanordnung nach Anspruch 16, wobei das Desinfektionsmittel oder das antimikrobielle Mittel ein Fluid oder ein Gel ist.

19. Spülspritzenanordnung nach Anspruch 16, wobei das Absorptionsmaterial ein Schaumstoff ist.

20. Spülspritzenanordnung nach Anspruch 19, wobei der Schaumstoff ein Polyurethan-Schaumstoff ist.

21. Spülspritzenanordnung nach Anspruch 16, wobei das Absorptionsmaterial ein Schwamm ist.

## Revendications

1. Ensemble seringue de rinçage comprenant:
un cylindre (20) incluant une paroi latérale ondulée (22) ayant une surface intérieure (23) définissant une chambre (24), une extrémité proximale fermée (26), une extrémité distale (28) incluant une paroi distale (29) avec un embout allongé (30) s'étendant distalement à partir de celle-ci ayant un passage (40) à travers elle en communication fluidique avec ladite chambre (24);
une quantité présélectionnée de fluide dans la chambre (24);
un collier (50) s'étendant depuis la paroi distale (29) du cylindre (20) et entourant l'embout allongé (30), le collier (50) incluant au moins une paroi latérale (51) ayant une surface intérieure (53) définissant un compartiment (52), une extrémité distale ouverte (54), une extrémité proximale (56) adjacente à la paroi distale (29) du cylindre (20); et
un élément de verrouillage (70) disposé sur une surface extérieure (55) de la paroi latérale ondulée (22) du cylindre (20);
**caractérisé en ce que**:
l'élément de verrouillage (70) comprend au moins une saillie (72) et au moins une cavité correspondante (74), la au moins une saillie (72) est disposée sur un segment de la paroi latérale ondulée (22) du cylindre (20) et la au moins une cavité correspondante (74) est disposée sur un segment opposé de la paroi latérale ondulée (22) du cylindre (20),
de sorte que la saillie 72 est en contact et s'engage avec la cavité 74 pour verrouiller l'extrémité proximale du cylindre à l'extrémité distale du cylindre, après que le fluide a été expulsé de la chambre 24 du cylindre 20
de sorte que l'élément de verrouillage (70) fournit un retour tactile, visuel et audible.

2. Ensemble seringue de rinçage selon la revendication 1, dans lequel le fluide est un fluide de rinçage.

3. Ensemble seringue de rinçage selon la revendication 2, dans lequel le fluide de rinçage est une solution saline, de l'héparine, de l'eau ou une combinaison de celles-ci.

4. Ensemble seringue de rinçage selon la revendication 1, dans lequel le compartiment (52) du collier (50) entoure l'embout allongé (30).

5. Ensemble seringue de rinçage selon la revendication 1, dans lequel la surface intérieure (23) du collier (50) a une pluralité de filetages (80).

6. Ensemble seringue de rinçage selon la revendication 5, dans lequel la pluralité de filetages sont adaptés au raccordement à un connecteur Luer femelle.

7. Ensemble seringue de rinçage selon la revendication 1, dans lequel la surface extérieure (55) du collier (50) a une pluralité de filetages (80).

8. Ensemble seringue de rinçage selon la revendication 5, dans lequel la pluralité de filetages (80) sont adaptés au raccordement à un connecteur Luer mâle.

9. Ensemble seringue de rinçage selon la revendication 1, dans lequel l'élément de verrouillage (70) minimise le reflux de solution dans le passage (40).

10. Ensemble seringue de rinçage selon la revendication 1, dans lequel l'élément de verrouillage (70) comprend des crans.

11. Ensemble seringue de rinçage selon la revendication 1, dans lequel l'élément de verrouillage (70) comprend un élément à encliquetage pour raccorder un segment de la paroi latérale ondulée (22) du cylindre (20) à un segment opposé de la paroi latérale ondulée (22) du cylindre (20).

12. Ensemble seringue de rinçage selon la revendication 1, comprenant en outre un joint amovible sur l'extrémité distale ouverte (28) du collier (50).

13. Ensemble seringue de rinçage selon la revendication 12, dans lequel le joint amovible est un joint pelable.

14. Ensemble seringue de rinçage selon la revendication 12, dans lequel le joint amovible comprend une partie arrière supérieure pelable en aluminium ou en film polymère multicouche.

15. Ensemble seringue de rinçage selon la revendication 12, dans lequel le joint amovible est thermoscellé ou scellé par induction à l'extrémité distale ouverte (28) du collier (50).

16. Ensemble seringue de rinçage selon l'une quelconque des revendications 1 à 15 comprenant en outre:
un matériau absorbant disposé dans le compartiment du collier;
un désinfectant ou un agent antimicrobien.

17. Ensemble seringue de rinçage selon la revendication 16, dans lequel l'agent désinfectant ou antimicrobien est choisi dans le groupe consistant en l'alcool isopropylique, l'éthanol, le 2-propanol, le butanol, le méthylparabène, l'éthylparabène, le propylparabène, le gallate de propyle, l'hydroxyanisole butylé (BHA), l'hydroxytoluène butylé, la t-butyl-hydroquinone, le chloroxylénol, la chlorhexidine, le diacétate de chlorhexidine, le gluconate de chlorhexidine, la povidone iodée, un alcool, l'alcool dichlorobenzylique, l'acide déhydroacétique, l'hexétidine, le triclosan, le peroxyde d'hydrogène, l'argent colloïdal, le chlorure de benzéthonium, le chlorure de benzalkonium, l'octénidine, un antibiotique et leurs mélanges.

18. Ensemble seringue de rinçage selon la revendication 16, dans lequel l'agent désinfectant ou antimicrobien est un fluide ou un gel.

19. Ensemble seringue de rinçage selon la revendication 16, dans lequel le matériau absorbant est une mousse.

20. Ensemble seringue de rinçage selon la revendication 19, dans lequel la mousse est une mousse de polyuréthane.

21. Ensemble seringue de rinçage selon la revendication 16, dans lequel le matériau absorbant est une éponge.
